**Europäisches Patentamt**

**European Patent Office**

**Office européen des brevets**

(19)

(11) Veröffentlichungsnummer : **0 401 454 B1**

## (12) EUROPÄISCHE PATENTSCHRIFT

(45) Veröffentlichungstag der Patentschrift :
**07.01.93 Patentblatt 93/01**

(51) Int. Cl.$^5$ : **A61K 6/06,** A61K 7/09,
A61K 7/135

(21) Anmeldenummer : **89810732.1**

(22) Anmeldetag : **27.09.89**

(54) **Haarpflegemittel zur Vermeidung von oxidativen Schädigungen des Haares.**

(30) Priorität : **16.03.89 CH 980/89**

(43) Veröffentlichungstag der Anmeldung :
**12.12.90 Patentblatt 90/50**

(45) Bekanntmachung des Hinweises auf die
Patenterteilung :
**07.01.93 Patentblatt 93/01**

(84) Benannte Vertragsstaaten :
**AT BE CH DE ES FR GB GR IT LI LU NL SE**

(56) Entgegenhaltungen :
**EP-A- 0 099 109**
**DE-A- 1 444 216**
**DE-A- 3 642 097**
**DE-C- 930 581**

(73) Patentinhaber : **MONO-COSMETIC S.A.**
**Bahnhofstrasse 11**
**CH-6340 Baar (CH)**

(72) Erfinder : **Bauer, Robert**
**Im Hirshalm 42**
**CH-4125 Riehen (CH)**

(74) Vertreter : **Feldmann, Clarence Paul et al**
**c/o Patentanwaltsbüro FELDMANN AG**
**Postfach Kanalstrasse 17**
**CH-8152 Glattbrugg (CH)**

Jouve, 18, rue Saint-Denis, 75001 PARIS

EP 0 401 454 B1

## Beschreibung

Die vorliegende Erfindung betrifft ein Haarpflegemittel zur Vermeidung von oxidativen Schädigungen des Haares durch Hydrogenperoxidreste.

Es ist bekannt, dass in menschlichen Haaren, besonders bei UV-Bestrahlung oder in feuchtem Zustand, Hydroperoxide gebildet werden. Es ist ferner bekannt, dass nach Wasserstoffperoxid- oder Persulfatbehandlungen Reste von Hydroperoxiden im Haar verbleiben, die auch durch intensives Spülen und Shampoonieren nicht entfernt werden können. Die im Haar vorhandenen Hydroperoxide bewirken nach einiger Zeit eine gewisse Haarbleichung, verbunden mit einer unerwünschten Haarschädigung, das heisst, das Haar wird stumpf und brüchig.

Eine augenfällige negative Beeinflussung des Haares wird sichtbar, wenn man die Haare unter Mitverwendung von Persulfaten bleicht. Die auf diese Weise gebleichten Haare lassen sich bei nachträglicher Färbung mit Oxidationsfarbstoffen, Nitroaminobenzolen oder Nitroaminophenolen nicht gleichmässig einfärben. Vielfach verändert sich auch die bei vorheriger Behandlung erzeugte Haarfarbe nachträglich in unerwünschter und unregelmässiger Weise. Dies beruht hauptsächlich darauf, dass sich nach Anwendung von Persulfaten und Wasserstoffperoxid, noch tagelang oder sogar nach Wochen Spuren des Peroxides auf dem Haar nachweisen lassen. (Zum Beispiel mittels Merckoquant Peroxid-Test der Firma Merck AG, Darmstadt, BRD).

Generell entsteht somit immer eine oxidative Schädigung des Haares durch reaktiven Sauerstoff ($H_2O_2$). Peroxid ist nach dem Färben, Colorieren, Blondieren und nach Behandlungen zum Fixieren der Dauerwellen immer vorhanden und nachweisbar.

Die Entfernung reaktiver Sauerstoffreste ($H_2O_2$) ist somit eine Voraussetzung für die Gesunderhaltung von chemisch behandeltem Haar.

Durch Behandlung mit Substanzen die Hydroperoxide zerstören wurde versucht, menschliche Haare von Hydroperoxidresten zu befreien, respektiv deren Bildung zu verhindern.

Ein nicht unwesentliches Problem stellte jedoch die Haar-und Hautverträglichkeit des Reduktionsmittels dar. Erschwerend ist insbesondere, dass solche Reduktionsmittel oftmals luft-, licht-, hitze- und feuchtigkeitsempfindlich sind. Ihre Lagerfähigkeit in wässerigen Lösungen ist folglich auch nur sehr begrenzt.

Die Erfindung stellt sich daher zur Aufgabe, ein Haarpflegemittel zur Vermeidung von oxidativen Schädigungen des Haares zu entwickeln, das gut haar- und hautverträglich ist und eine erheblich verbesserte Lagerfähigkeit aufweist.

Der Einsatz von Ascorbinsäure in Haarfärbemittel ist an sich bekannt. So beschreibt die DE-A-1'444'216 ein flüssiges Haarfärbemittel, welches L-Ascorbinsäure enthält. Diese dient hier als Reduktionsmittel, um das sonst unstabile flüssige Mittel haltbar zu machen. Auch das Oxidationshaarfärbemittel gemäss der DE-A-36'42'097 enthält Ascorbinsäure als Stabilisationsmittel. Erst der Zusatz der Ascorbinsäure gewährleistet, dass nicht bereits während der Herstellung und des Abfallens eine Zersetzung eintritt.

Schliesslich beschreibt das Haarnachbehandlungsmittel gemäss der DE-C-930'581 die Verwendung der Ascorbinsäure neben der Salicylsäure zum hier beschriebenen Zweck. Das hier in nicht-pulverförmiger Form vorliegende Mittel hat somit nur eine begrenzte Lagerungsstabilität.

Diese Aufgabe erfüllt ein Haarpflegemittel mit den Merkmalen des Patentanspruches 1.

Versuche haben gezeigt, dass Ascorbinsäure besonders geeignet ist, als haar- und hautverträgliches Reduktionsmittel, die im Haar nach dem Färben, Colorieren, Blondieren und nach Behandlungen zum Fixieren von Dauerwellen vorhandenen Hydroperoxidreste zu reduzieren. Der gewichtsmässige Anteil der Ascorbinsäure, die in kristalliner Form verwendet wird, kann fast beliebig variiert werden. Vorzugsweise beträgt der gewichtsmässige Anteil der Ascorbinsäure 5 bis 50 %.

Die Lagerfähigkeit und Stabilität des Haarpflegemittels wurde erreicht durch die Verwendung von umhüllter Ascorbinsäure. Auf dem Markt ist beispielsweise Silicon-umhüllte Ascorbinsäure unter der Typenbezeichnung SC und mit Aethylcellulose umhüllte Ascorbinsäure unter der Typenbezeichnung EC, beide von der Firma F. Hoffmann-La Roche & Co. AG, Basel/Schweiz, erhältlich.

Ohne die Wirkung der Ascorbinsäure zu mindern, wurden hier bekannte pulverförmige, wasserlösliche kationenaktive Verbindungen zugesetzt. Dadurch werden alle gewünschten Effekte eines Nachbehandlungsmittels wie, Nass- und Trockenkämmbarkeitsverbesserung, Glanz, Geschmeidigkeit und allgem. Pflege des Haares erzielt. Im speziellen kommen hierfür quaternäre Ammoniumverbindungen wie Cetyltrimethylammoniumbromid in Frage. Aber auch Copolymere von Hydroxyaethylcellulose und einer quaternären Ammoniumverbindung sind gut geeignet. Beispielsweise bietet die Firma National Starch and Chemical Corporation, Bridgewater, New Jersey, USA unter der Bezeichnung Celquat ® L 200 ein Copolymer von Hydroxyaethylcellulose und Diallyldimethylammoniumchlorid an, das sich gut eignet.

Zur Bindung der in Spuren vorhandenen Schwermetallen kann man das Haarpflegemittel mit einem Komplexbildner versehen, der seine Wirkung auch auf stark kalkhaltiges Wasser ausübt. Ein besonders geeigneter Komplexbildner ist beispielsweise Aethylendiamintetraessigsäure (EDTA) oder deren Natriumsalze.

Die Wasserlöslichkeit der quaternären Ammoniumverbindungen wird durch den Zusatz von Natriumhydrogencarbonat, welches unter Brausen mit den Säuren reagiert, beschleunigt. Der für diese Reaktion erforderliche Anteil an Ascorbinsäure kann durch eine preiswertere Säure, beispielsweise Wein-oder Zitronensäure ersetzt werden.

Eine Klumpenbildung des Haarpflegemittels in Pulverform lässt sich durch Zugabe von hochdispersem, amorphem Siliciumdioxid, welches unter der Bezeichnung Aerosol ® von der Firma Degussa AG, Frankfurt, BRD angeboten wird, vermeiden.

Falls erwünscht lässt sich auch Parfüm in geringer Dosis beimischen.

Als Füllstoffe werden Zucker oder Zuckeralkohole zugefügt, beispielsweise verwendet man D(+) Glucose wasserfrei, welche wegen ihrer ausgezeichneten Hautverträglichkeit besonders geeignet sind. Der pH-Wert des mit Wasser zu mischenden Haarpflegemittels kann beliebig eingestellt werden, vorzugsweise jedoch so, dass ein pH-Wert zwischen 5,0 und 8,5 der Lösung resultiert. Bei mit Wasserstoffperoxid behandeltem Haar sind bei einer Nachbehandlung mit dem erfindungsgemässen Haarpflegemittel bereits nach 2-5 Minuten keine Hydrogenperoxidreste mehr nachweisbar. Auch Oxidationshaarfärbungen, welche bei mit Persulfatverbindungen gebleichten Haaren durchgeführt werden, ergeben nach einer Zwischenbehandlung mit dem beanspruchten Haarpflegemittel gleichmässige Färbeergebnisse.

Nachfolgend sind drei Beispiele (a-c) erfindungsgemässer Rezepturen des Haarpflegemittels aufgeführt. Sämtliche Mengenangaben sind in Gewichtsprozenten angegeben:

|  |  | % |
|---|---|---|
| a) | Aethylendiamintetraessigsäure (EDTA) | 0,100 |
|  | Celquat® L 200 | 4,000 |
|  | Aerosil® 200 | 0,500 |
|  | Cetyltrimethylammoniumbromid gepulvert | 15,000 |
|  | Ascorbinsäure Typ EC | 15,000 |
|  | Natriumhydrogencarbonat reinst wasserfrei | 7,500 |
|  | Sorbit gepulvert wasserfrei | 57,900 |
|  |  | 100,000 |

|  |  | % |
|---|---|---|
| b) Aethylendiamintetraessigsäure Na   (EDTA Na$_4$) | | 0,100 |
| Celquat® L 200 | | 5,000 |
| Aerosil® 300 | | 0,600 |
| Cetyltrimethylammoniumbromid gepulvert | | 12,000 |
| Weinsäure gepulvert reinst | | 5,000 |
| Ascorbinsäure Typ SC | | 5,000 |
| Natriumhydrogencarbonat reinst wasserfrei | | 8,500 |
| Mannit gepulvert wasserfrei | | 63,800 |
| | | 100,000 |

|  |  | % |
|---|---|---|
| c) Aethylendiamintetraessigsäure Na   (EDTA Na$_2$) | | 0,100 |
| Celquat® L 200 | | 5,000 |
| Cetyltrimethylammoniumbromid gepulvert | | 15,000 |
| Weinsäure gepulvert reinst | | 9,000 |
| Ascorbinsäure Typ SC | | 6,000 |
| Natriumhydrogencarbonat reinst wasserfrei | | 12,500 |
| Glucose gepulvert wasserfrei | | 51,630 |
| Aerosil® 200 | | 0,700 |
| Parfüm | | 0,070 |
| | | 100,000 |

Die Anwendung obiger Rezepturen ist einfach, wie nachfolgende Beispiele zeigen. Das in luftdichte Beutel abgepackte Haarpflegemittel wird kurz vor Gebrauch im Verhältnis 1:3-1:4 in kaltem oder lauwarmem Wasser unter Brausen gelöst.

Beispiele :

1.) Menschliche Haare werden mit üblichen Thioglykolatlösungen dauergewellt, mit Wasserstoffperoxid fixiert, gespült und mit einer Lösung der unter a-c genannten Beispiele etwa 2-5 Minuten nachbehandelt.
2.) Menschliche Haare werden unter Verwendung von Ammonium- und/oder Kaliumpersulfat und Wasserstoffperoxid in üblicher Weise gebleicht und mit einer Lösung der unter a-c genannten Beispiele 2-5 Minuten nachbehandelt.
3.) Menschliche Haare werden mit Haarfärbemitteln auf Basis von Oxidationsfarbstoffen unter Mitverwendung von Wasserstoffperoxid oder wasserstoffperoxid-abgebenden Substanzen, zum Beispiel Percarbamid Wasserstoffperoxid Harnstoff, in üblicher Weise gefärbt und mit einer Lösung der unter a-c genannten Beispiele 2-5 Minuten nachbehandelt.

Bei den nach den Beispielen 1-3 nachbehandelten Haaren lassen sich keine Peroxidrückstände mehr nachweisen. Die nach Beispiel 3 behandelten Haare lassen sich mit Oxidationshaarfarben schön gleichmässig einfärben.

Zur Herstellung werden alle Komponenten durch ein 0,5 mm V$_2$A-Stahl Sieb gesiebt (Siliciumdioxid und Parfum werden separat vorgemischt) und anschliessend in einem geeigneten Pulvermischer homogen vermischt.

## Patentansprüche

1. Haarpflegemittel zur Vermeidung von oxidativen Schädigungen des Haares durch Hydrogenperoxid, welches als wasserlösliches pulver- oder tablettenförmiges Produkt vorliegt und mindestens die folgenden Komponenten umfasst:
   - wasserlösliche, pulverförmige kationenaktive Verbindungen,
   - ein Copolymer von Hydroxyaethylcellulose und einer quaternären Ammoniumverbindung,
   - Natriumhydrogencarbonat zur verbesserten Wasserlöslichkeit,
   - sowie als haar- und hautverträgliches Reduktionsmittel ummantelte Ascorbinsäure.

2. Haarpflegemittel nach Anspruch 1, dadurch gekennzeichnet, dass es 5-50 Gewichtsprozente Ascorbinsäure enthält.

3. Haarpflegemittel nach Anspruch 1, dadurch gekennzeichnet, dass die Ascorbinsäure mit Silicon oder Aethylcellulose umhüllt ist.

4. Haarpflegemittel nach Anspruch 1, dadurch gekennzeichnet, dass es als kationenaktive Verbindungen quaternäre Ammoniumverbindungen enthält.

5. Haarpflegemitel nach Anspruch 4, dadurch gekennzeichnet, dass es als quaternäre Ammoniumverbindung Cetyltrimethylammoniumbromid enthält.

6. Haarpflegemittel nach Anspruch 1, dadurch gekennzeichnet, dass es ein Copolymer von Hydroxyaethylcellulose und Diallyldimethylammoniumchlorid enthält.

7. Haarpflegemittel nach Anspruch 1, dadurch gekennzeichnet, dass es als Komplexbildner, Aethylendiamintetraessigsäure (EDTA) oder deren Natriumsalze enthält.

8. Haarpflegemittel nach Anspruch 1, dadurch gekennzeichnet, dass es zur Reaktion mit dem Natriumhydrogencarbonat noch Wein- oder Zitronensäure enthält.

9. Haarpflegemittel nach Anspruch 1, dadurch gekennzeichnet, dass es als Füllstoff Zucker oder Zuckeralkohole enthält.

10. Haarpflegemittel nach Anspruch 9, dadurch gekennzeichnet, dass es D(+) Glucose wasserfrei enthält.

11. Haarpflegemittel nach Anspruch 1, dadurch gekennzeichnet, dass es hochdisperses, amorphes Siliciumdioxid enthält.

12. Haarpflegemittel nach Anspruch 1, dadurch gekennzeichnet, dass es Parfüm enthält.

## Claims

1. A hair care agent for preventing oxidation damage to the hair by hydrogen peroxide, said agent taking the form of a water-soluble pulverulent or tablet-shaped product and comprising at least the following components:
   - water-soluble pulverulent cation-active compounds,
   - a copolymer of hydroxyethyl cellulose and a quaternary ammonium compound,
   - sodium hydrogen carbonate for improved water solubility,

- a coated ascorbic acid as a hair and skin compatible reducing agent.

2. A hair care agent according to claim 1, characterized in that it contains 5-50% by weight of ascorbic acid.

3. A hair care agent according to claim 1, characterized in that the ascorbic acid is coated with silicon or ethyl cellulose.

4. A hair care agent according to claim 1, characterized in that it contains quaternary ammonium compounds as the cation-active compounds.

5. A hair care agent according to claim 4, characterized in that it contains cetyltrimethyl ammonium bromide as a quaternary ammonium compound.

6. A hair care agent according to claim 1, characterized in that it contains a copolymer of hydroxy ethyl cellulose and diallyldimethyl ammonium chloride.

7. A hair care agent according to claim 1, characterized in that it contains ethylene diamine tetraacetic acid (EDTA) or its sodium salts as a complex former.

8. A hair care agent according to claim 1, characterized in that it also contains tartaric acid or citric acid for reaction with the sodium hydrogen carbonate.

9. A hair care agent according to claim 1, characterized in that it contains sugar or sugar alcohols as fillers.

10. A hair care agent according to claim 9, characterized in that it contains anhydrous D (+) glucose.

11. A hair care agent according to claim 1, characterized in that it contains highly disperse amorphous silicon dioxide.

12. A hair care agent according to claim 1, characterized in that it contains perfume.

**Revendications**

1. Produit de soin capillaire destiné à éviter des endommagements par oxydation des cheveux causés par du peroxyde d'hydrogène, qui se présente sous la forme d'un produit en poudre ou en comprimés soluble dans l'eau et qui comporte au moins les composants suivants :
   - des composés tensioactifs cationiques pulvérulents, solubles dans l'eau,
   - un copolymère d'hydroxyéthylcellulose et d'un composé d'ammonium quaternaire,
   - du bicarbonate de sodium pour améliorer la solubilité dans l'eau,
   - ainsi que de l'acide ascorbique enrobé, comme réducteur compatible avec les cheveux et la peau.

2. Produit de soin capillaire suivant la revendication 1, caractérisé en ce qu'il contient 5 à 50 % en poids d'acide ascorbique.

3. Produit de soin capillaire suivant la revendication 1, caractérisé en ce que l'acide ascorbique est enrobé par de la silicone ou de l'éthylcellulose.

4. Produit de soin capillaire suivant la revendication 1, caractérisé en ce qu'il contient, comme composés tensioactifs cationiques, des composés d'ammonium quaternaires.

5. Produit de soin capillaire suivant la revendication 4, caractérisé en ce qu'il contient, comme composé d'ammonium quaternaire, du bromure de cétyltriméthyl-ammonium.

6. Produit de soin capillaire suivant la revendication 1, caractérisé en ce qu'il contient un copolymère d'hydroxyéthylcellulose et de chlorure de diallyldiméthyl-ammonium.

7. Produit de soin capillaire suivant la revendication 1, caractérisé en ce qu'il contient, comme complexant, de l'acide éthylènediaminetétraacétique (EDTA) ou des sels de sodium de celui-ci.

8. Produit de soin capillaire suivant la revendication 1, caractérisé en ce qu'il contient encore de l'acide tartrique ou citrique pour la réaction avec le bicarbonate de sodium.

9. Produit de soin capillaire suivant la revendication 1, caractérisé en ce qu'il contient, comme charge, du sucre ou des alcools de sucre.

10. Produit de soin capillaire suivant la revendication 9, caractérisé en ce qu'il contient à l'état anhydre du D(+) glucose.

11. Produit de soin capillaire suivant la revendication 1, caractérisé en ce qu'il contient du dioxyde de silicium amorphe, hautement dispersé.

12. Produit de soin capillaire suivant la revendication 1, caractérisé en ce qu'il contient du parfum.